# EUROPÄISCHE PATENTANMELDUNG

(11) **EP 2 005 922 A1**
(43) Veröffentlichungstag der Anmeldung: **24.12.2008**
(21) Anmeldenummer: 08010151.2
(22) Anmeldetag: 04.06.2008
(51) Int. Cl.: A61F 7/00

(54) **Vorrichtung zur Wärmeabgabe, insbesondere Wärmeflasche**

(30) Priorität: 06.06.2007 DE 202007007984 U
(71) Anmelder: Schodde, Burkhard, 26188 Edewecht (DE); Konarek, Thorsten, 26188 Edewecht (DE)
(72) Erfinder: Schodde, Burkhard, 26188 Edewecht (DE); Konarek, Thorsten, 26188 Edewecht (DE)
(74) Vertreter: Jabbusch, Matthias

(57) **Zusammenfassung**

Die Erfindung betrifft eine Vorrichtung zur Wärmeabgabe, insbesondere eine Wärmflasche mit einem Aufnahmebehälter für ein wärmespeicherndes, flüssige Medium, wobei im Inneren des Aufnahmebehälters wenigstens ein elektrisches Heizelement angeordnet ist.

Der Erfindung liegt die Aufgabe zugrunde, eine solche Vorrichtung so weiterzuentwickeln, daß die Verwendung vereinfacht ist und die Benutzung derselben eine größere Sicherheit aufweist.

Diese Aufgabe ist dadurch gelöst, daß in der Behälterwandung des Aufnahmebehälters ein Kontaktstecker angeordnet ist, der mit dem Heizelement stromleitend verbunden ist und daß der die Behälterwandung durchdringende Kontaktstecker mit einer Kontaktsteckeraufnahme einer externen, an eine Stromquelle anzuschließenden Aufstellplatte in stromleitende Verbindung bringbar ist.

## Beschreibung

Die Erfindung bezieht sich auf eine Vorrichtung zur gezielten Wärmeabgabe, insbesondere eine Wärmflasche, mit einem Aufnahmebehälter für ein wärmespeicherndes, flüssiges Medium, wobei im Inneren des Aufnahmebehälters wenigstens ein elektrisches Heizelement angeordnet ist.

Bekannte Vorrichtungen zur Wärmeabgabe sind unter anderem Wärmflaschen, welche beispielsweise zur äußeren Wärmebehandlung von Körperpartien eines menschlichen Körpers und damit zur Verbesserung des Wohlbefindens einer zu behandelnden Person eingesetzt werden.

Aus der Druckschrift DE 8315271 U1 oder der Druckschrift DE 20 2007 003 992 U1 ist eine derartige Vorrichtung zur Wärmeabgabe bekannt, welche im Inneren ihres Aufnahmebehälters wenigstens ein elektrisches Heizelement zur Erwärmung des Mediums aufweist. Das Heizelement ist dabei insbesondere mit einem im Bereich des Einfüllstutzens für das Medium angeordneten Kontaktstecker elektrisch leitend verbunden. An dem Kontaktstecker wird dann zur Stromversorgung der Vorrichtung ein an sich bekanntes Stromkabel stromleitend angeschlossen, welches nach dem Erwärmen des Mediums wieder abgezogen werden muß.

Bei der Verwendung der bekannten Vorrichtungen können Schwierigkeiten und Gefahren durch das relativ umständlich an dem Kontaktstecker anzuschließende Stromkabel oder nach der Erwärmung des wärmespeichemden Mediums durch das nicht abgezogene Stromkabel auftreten. Des weiteren ist darauf zu achten, daß während des Aufwärmens des Mediums der Aufnahmebehälter stets auf einer wärmeunempfindlichen Unterlage aufliegt, um etwaige Beschädigungen an Mobiliar oder Einrichtungsgegenständen zu vermeiden.

Der Erfindung liegt die Aufgabe zugrunde, eine Vorrichtung zur gezielten Wärmeabgabe zu schaffen, bei deren Verwendung eine vereinfachte und zugleich sichere Handhabung gegeben ist.

Die Lösung der Aufgabe erfolgt erfindungsgemäß durch eine Vorrichtung mit den Merkmalen des Schutzanspruches 1. Vorteilhafte Weiterbildungen und Ausgestaltungen der Erfindung sind in den Ansprüche 2 bis 7 angegeben.

Bei einer Vorrichtung zur Wärmeabgabe, insbesondere einer Wärmflasche, mit einem Aufnahmebehälter für ein wärmespeichemdes, flüssiges Medium, wobei im Inneren des Aufnahmebehälters wenigstens ein elektrisches Heizelement angeordnet ist, ist nach der Erfindung vorgesehen, daß in der Behälterwandung des Aufnahmebehälters ein Kontaktstecker angeordnet ist, welcher mit dem Heizelement stromleitend verbunden ist, und daß der die Behälterwandung durchdringende Kontaktstecker mit einer Kontaktsteckeraufnahme einer externen, an einer Stromquelle anzuschließenden Aufstellplatte in stromleitende Verbindung bringbar ist.

Mit Hilfe eines in der Behälterwandung des Aufnahmebehälters angeordneten Kontaktsteckers und einer an einer externen Aufstellplatte ausgebildeten Kontaktsteckeraufnahme läßt sich deren stromleitende Verbindung auf vorteilhafte Weise herstellen, wodurch ebenfalls die Stromversorgung des Heizelementes mit Vorteil gewährleistet ist. Die Vorrichtung zur Wärmeabgabe wird dazu einfach mit der an einer Stromquelle anzuschließenden Aufstellplatte durch Aufstecken elektrisch verbunden. Auf den Einsatz eines mit dem Kontaktstecker in der Regel aufwendig zu verbindenden Stromkabels kann somit mit Vorteil verzichtet werden. Nach Erreichen eines vorbestimmten Temperaturniveaus des wärmespeichemden Mediums im Aufnahmebehälter, welches sowohl flüssig als auch zähflüssig, gelartig, also viskos sein kann, läßt sich dann der Aufnahmebehälter der Vorrichtung von der Aufstellplatte einfach abnehmen, worauf er als Wärmflasche insbesondere zur Wärmebehandlung einer Person eingesetzt werden kann. Somit dient die Aufstellplatte gleichzeitig als Ablagefläche für den Aufnahmebehälter der Vorrichtung, weshalb der aufgeheizte Aufnahmebehälter nicht mit einem insbesondere wärmeempfindlichen Untergrund in Kontakt gelangt. Zu diesem Zweck kann die Aufstellplatte entsprechend an die äußere Kontur des Aufnahmebehälters angepaßte Abmessungen aufweisen. Der verwendete Aufnahmebehälter kann beispielsweise durch eine das wärmespeichernde Medium vollständig einschließende Behälterwandung ausgebildet sein, welcher keinen direkten Zugang zum Inneren des Aufnahmebehälters aufweist.

Das Heizelement ist vorzugsweise als Flächenheizkörper ausgebildet. Das Heizelement weist dementsprechend eine vorteilhaft große Wärmeübertragungsfläche auf, was zur Folge hat, daß sich das flüssige oder gelartige Medium nahezu gleichmäßig erwärmen läßt und somit die Übertragungsverluste gering gehalten werden können. Um einen direkten Kontakt des Flächenheizkörpers mit der Innenseite der Behälterwandung des Aufnahmebehälters und dadurch mögliche Beschädigungen derer zu vermeiden, kann der Flächenheizkörper in vorbestimmten Bereichen mit Abstandshaltern ausgerüstet sein.

Darüber hinaus ist das Heizelement mit einem den Stromfluß für das Heizelement schaltenden Temperatursensor ausgerüstet, welcher nach Erreichen eines bestimmten Temperaturniveaus innerhalb des Aufnahmebehälters den Stromfluß für das Heizelement unterbricht und somit die Wärmezufuhr des Heizelementes zum wärmespeichemden Medium stoppt. Über den Temperatursensor läßt sich demzufolge ein Sieden des Mediums, was zum Beispiel bei Wasser etwa bei 100°C erfolgt, innerhalb des Behälters verhindern. Somit kann die Entstehung eines Überdruckes im Aufnahmebehälter vermieden und einer möglicherweise daraus resultierenden Beschädigung der Behälterwandung entgegengewirkt werden.

Der Temperatursensor weist beispielsweise einen Bimetallstreifen auf, mit Hilfe dem eine konstruktiv einfache Möglichkeit zur gleichzeitigen Erfassung eines bestimmten Temperaturniveaus im Aufnahmebehälter und der Unterbrechung der Stromzufuhr für das Heizelement möglich ist. Die zur Ausbildung des Bimetallstreifens eingesetzten, miteinander in Kontakt gebrachten metallischen Werkstoffe können je nach gewählter Werkstoffkombination dann die Stromunterbrechung beim Erreichen jeweils unterschiedlicher Temperaturen bewirken. Es ist selbstverständlich auch möglich, eine elektronische Meßwerterfassung zur Temperaturmessung und der Stromunterbrechung am Heizelement vorzusehen.

Nach einer Weiterbildung der Erfindung ist vorgesehen, daß der Aufnahmebehälter mit einem Einfüllstutzen für das wärmespeichernde, flüssige bzw. gelartige Medium versehen ist. Nach einer vorbestimmten Betriebsdauer kann es unter Umständen notwendig sein, einen Austausch des im Aufnahmebehälter befindlichen Mediums vorzunehmen, um stets einen sicheren Betrieb der erfindungsgemäßen Vorrichtung gewährleisten zu können. Des weiteren kann durch das sich ständig wiederholende Erhitzen des Mediums ein Flüssigkeitsverlust im Aufnahmebehälter auftreten, so daß sich das Medium über den Einfüllstutzen vorteilhaft einfach nachfüllen läßt, um mit Vorteil einen Mindestflüssigkeitsstand innerhalb des Aufnahmebehälters sicherzustellen. Der Einfüllstutzen kann beispielsweise im Seitenbereich des Aufnahmebehälters ausgebildet und über einen Schraubstopfen abdichtend verschlossen sein.

Der Werkstoff für den Aufnahmebehälter kann ein elastische Eigenschaften aufweisender Gummi sein, wodurch ein vorteilhafter Einsatz bei der Wärmebehandlung der Körperpartien gewährleistet ist. Die elastische bzw. flexible Ausgestaltung der Behälterwandung ermöglicht eine optimale Anpassung an die Form der jeweils zu behandelnden Körperpartie, worüber ein größtmöglicher Wärmeeintrag in die zu behandelnde Zone sichergestellt ist.

Eine alternative Ausgestaltungsform sieht vor, daß der Werkstoff für den Aufnahmebehälter ein hartelastische Eigenschaften aufweisender Kunststoff ist. Insbesondere bei einer als Bettwärmer ausgebildeten erfindungsgemäßen Vorrichtung zur Wärmeabgabe kann es von Vorteil sein, wenn zur Ausbildung der Behälterwandung ein hartelastischer Kunststoff zum Einsatz kommt. Mittels solcher Kunststoffe lassen sich jeweils relativ große und zugleich formstabile Aufnahmebehälter ausgestalten, welche, aufgrund der relativ großen Menge des darin aufgenommenen Mediums, eine entsprechend lange Wärmeabgabe ermöglichen. Dabei ist darauf zu achten, daß das durch den Aufnahmebehälter aufgenommene Medium jedoch ein leicht handhabbares Volumen aufweist.

Ein Ausführungsbeispiel einer erfindungsgemäßen Vorrichtung, aus dem sich weitere erfinderische Merkmale ergeben, ist in der Zeichnung dargestellt. Es zeigen:
- Fig. 1:: eine Ansicht einer Vorrichtung zur gezielten Wärmeabgabe, und
- Fig. 2:: eine Ansicht der Vorrichtung nach Figur 1 in Verbindung mit einer Stromversorgungseinrichtung.

Mit 1 ist eine Wärmflasche bezeichnet, die zur gezielten Wärmeabgabe beispielsweise auf Partien des menschlichen Körpers verwendet wird. Die Wärmflasche 1 weist einen Aufnahmebehälter 2 für ein darin aufzunehmendes wärmespeichemdes, flüssiges Medium auf, das die gespeicherte Wärmeenergie über die Behälterwandung 3 des Aufnahmebehälters 2 abgibt. Innerhalb des Aufnahmebehälters ist ein nicht dargestelltes Heizelement angeordnet, welches das Medium im Inneren des Aufnahmebehälters aufheizt. Um die Stromversorgung des Heizelementes im Inneren der erfindungsgemäßen Wärmflasche zu gewährleisten, ist in der Behälterwandung 3 des Aufnahmebehälters 2 ein Kontaktstecker 4 angeordnet, welcher stromleitend mit dem Heizelement verbunden ist. Der Kontaktstecker 4 ist derart in der Behälterwandung integriert, daß dieser nahezu eben mit der Peripherie des Aufnahmebehälters abschließt. Der Aufnahmebehälter 2 weist des weiteren einen Einfüllstutzen 5 auf, über den der Austausch des wärmespeichemden Mediums bzw. ein Nachfüllen des Mediums möglich ist. Als Werkstoff für den Aufnahmebehälter 2 der dargestellten Wärmflasche 1 ist vorzugsweise ein elastische Eigenschaften aufweisender Gummi vorgesehen, der bei der Wärmebehandlung von Körperpartien eine vorteilhafte Anpassung an die Oberflächenform gewährleistet.

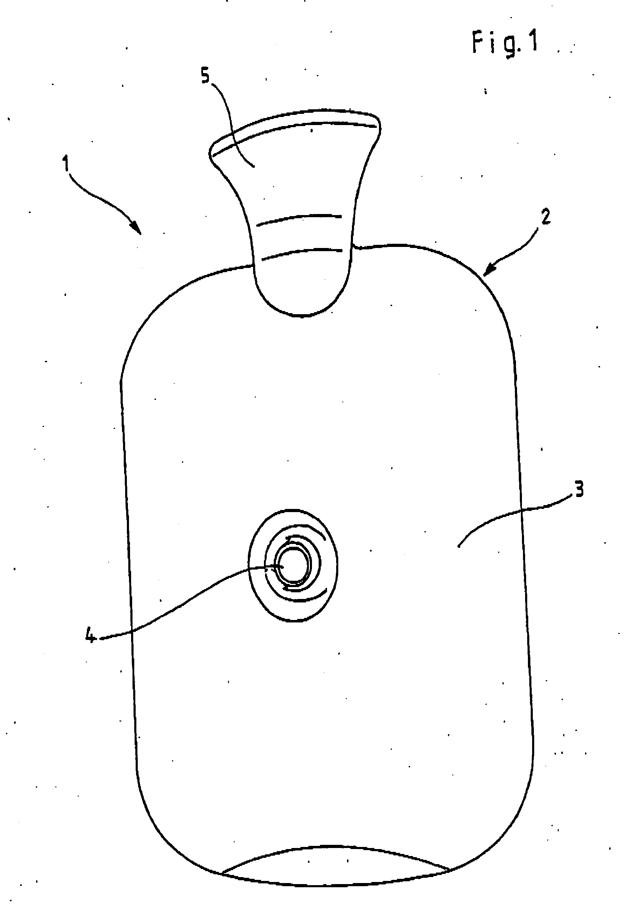

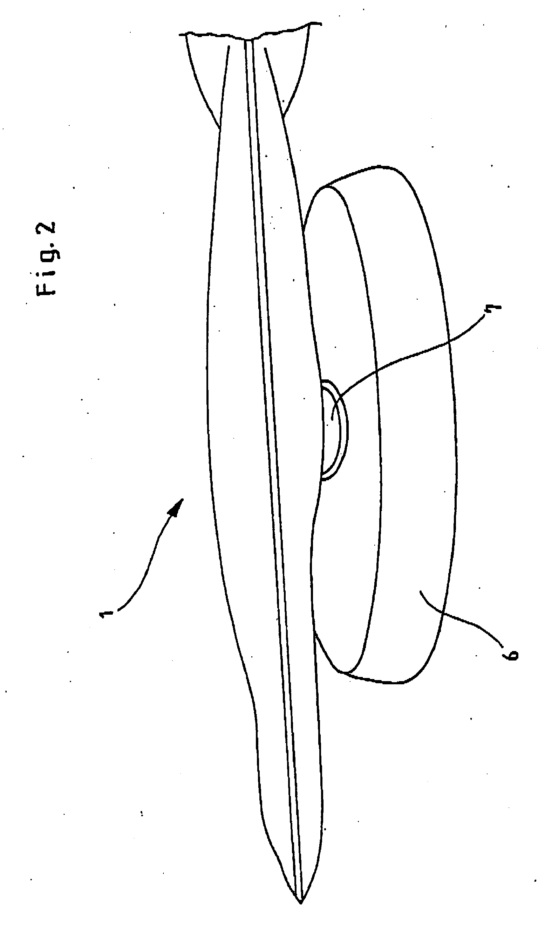

In Fig. 2 ist die erfindungsgemäße Wärmflasche nach Fig. 1 in Kombination mit einer Aufstellplatte 6 eines handelsverfügbaren Wasserkochers gezeigt. Die Aufstellplatte 6 weist für eine stromleitende Verbindung mit der Wärmflasche 1 eine mit deren Kontaktstecker 4 korrespondierende Kontaktsteckeraufnahme 7 auf, worüber die Stromversorgung der erfindungsgemäßen Wärmflasche erfolgt.

## Patentansprüche

1. Vorrichtung zur Wärmeabgabe, insbesondere Wärmflasche, mit einem Aufnahmebehälter für ein wärmlespeicherndes, flüssiges Medium, wobei im Inneren des Aufnahmebehälters wenigstens ein elektrisches Heizelement angeordnet ist,
**dadurch gekennzeichnet,**
**daß** in der Behälterwandung (3) des Aufnahmebehälters (2) ein Kontaktstecker (4) angeordnet ist, welcher mit dem Heizelement stromleitend verbunden ist, und
**daß** der die Behälterwandung (3) durchdringende Kontaktstecker (4) mit einer Kontaktsteckeraufnahme (7) einer externen, an eine Stromquelle anzuschließenden Aufstellplatte (6) in stromleitende Verbindung bringbar ist.

2. Vorrichtung nach Anspruch 1, **dadurch gekennzeichnet, daß** das Heizelement als Flächenheizkörper ausgebildet ist.

3. Vorrichtung nach Anspruch 1 oder 2, **dadurch gekennzeichnet, daß** das Heizelement mit einem den Stromfluß für das Heizelement schaltenden Temperatursensor ausgerüstet ist.

4. Vorrichtung nach Anspruch 3, **dadurch gekennzeichnet, daß** der Temperatursensor einen Bimetallstreifen aufweist.

5. Vorrichtung nach einem der Ansprüche 1 bis 4, **dadurch gekennzeichnet, daß** der Aufnahmebehälter (2) mit einem Einfüllstützen (5) versehen ist.

6. Vorrichtung nach einem der Ansprüche 1 bis 5, **dadurch gekennzeichnet, daß** der Werkstoff für den Aufnahmebehälter (2) ein elastische Eigenschaften aufweisender Gummi ist.

7. Vorrichtung nach einem der Ansprüche 1 bis 6, **dadurch gekennzeichnet, daß** der Werkstoff für den Aufnahmebehälter (2) ein hartelastische Eigenschaften aufweisender Kunststoff ist.
